(19) Europäisches Patentamt
European Patent Office
Office européen des brevets

(11) Veröffentlichungsnummer: 0 342 363
A2

(12) # EUROPÄISCHE PATENTANMELDUNG

(21) Anmeldenummer: 89106785.2

(22) Anmeldetag: 15.04.89

(51) Int. Cl.4: C12N 1/20 , C12P 15/00 , C07C 49/727 , C07D 303/32 , A61K 31/12 , A61K 31/335

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4201, DSM 4202.

Patentansprüche für folgende Vertragsstaaten: ES + GR.

(30) Priorität: 26.04.88 DE 3813967

(43) Veröffentlichungstag der Anmeldung: 23.11.89 Patentblatt 89/47

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Grabley, Susanne, Dr.
Hölderlinstrasse 7
D-6240 Königstein/Taunus(DE)
Erfinder: Wink, Joachim, Dr.
Bieberer Strasse 133
D-6050 Offenbach(DE)
Erfinder: Giani, Carlo, Dr.
Hedderichstrasse 69
D-6000 Frankfurt am Main 70(DE)
Erfinder: Seibert, Gerhard, Prof. Dr.
Gläserweg 21
D-6100 Darmstadt(DE)
Erfinder: Raether, Wolfgang, Dr.
Falkensteinstrasse 6
D-6072 Dreieich(DE)
Erfinder: Dobreff, Susanne
Otto-Lauffer-Strasse 2
D-3400 Göttingen(DE)
Erfinder: Zeeck, Axel, Prof. Dr.
Brüder-Grimm-Allee 22
D-3400 Göttingen(DE)

(54) **Neue Angucyclinone aus Streptomyceten, Verfahren zu ihrer Herstellung und ihre Verwendung.**

(57) Mit Hilfe eines Stammes aus der Gattung Streptomyces können neue Angucyclinone mit therapeutischer Wirkung hergestellt werden.

EP 0 342 363 A2

## Neue Angucyclinone aus Streptomyceten, Verfahren zu ihrer Herstellung und ihre Verwendung

Es ist bekannt, daß Streptomyces spec. unter herkömmlichen Kulturbedingungen ein Angucyclinon mit Namen Ochromycinon synthetisiert [Bowie J.H., Johnson A.W. Tetrahedron Letters 16, 1449 (1967)].

Es wurde nun überraschend gefunden, daß Streptomyces spec. DSM 4201 und DSM 4202 neben Ochromycinon neue Angucyclinone mit antimikrobieller Wirkung und Aktivität gegen Protozoen bilden.

Die Erfindung betrifft somit:

1. Eine Verbindung der allgemeinen Formel I,

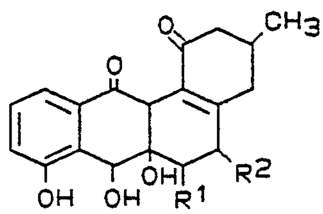

I

in der unabhängig voneinander $R^1$ Hydroxyl und $R^2$ Wasserstoff sein kann oder $R^1$ und $R^2$ zusammen mit den tragenden C-Atomen einen Oxiranring darstellen, sowie die an den in der Formel I aufgeführten Hydroxylgruppen derivatisierten ($C_1$bis$C_5$)-Acyloxyverbindungen.

2. Ein Verfahren zur Herstellung der Verbindung der allgemeinen Formel I, das dadurch gekennzeichnet ist, daß

a) Streptomyces spec. DSM 4201 und DSM 4202 kultiviert werden, bis sich die Verbindung der allgemeinen Formel I im Kulturmedium anhäuft und

b) gegebenenfalls die Verbindung isoliert und acyliert wird.

Die Erfindung wird im folgenden detailliert beschrieben, insbesondere in ihren bevorzugten Ausführungsformen. Ferner wird die Erfindung in den Patentansprüchen definiert.

Die Verbindung der allgemeinen Formel I kann mit Hilfe von Streptomyces spec. DSM 4201 und DSM 4202 hergestellt werden. Die Stämme wurden am 5.8.1987 bei der Deutschen Sammlung von Mikroorganismen unter der angegebenen Nummer nach den Bedingungen des BudapesterVertrages hinterlegt.

Streptomyces spec. DSM 4201 und DSM 4202 haben folgende charakteristische Merkmale:

| | |
|---|---|
| Sporenfarbe:<br>Sporenkette:<br>Sporenoberfläche:<br>Melaninbildung: | grau<br>Retinaculum apertum<br>glatt<br>negativ |
| Pigmentbildung: | |
| Substratmycel: | Endo: negativ<br>Exo: negativ |
| Luftmycel: | Endo: negativ<br>Exo: negativ |
| Zucker- und Zuckeralkoholverwertung: | Arabinose, Xylose, Rhamnose, Raffinose, Mannit, Inositol, Fructose, Saccharose. |

Anstelle von Streptomyces spec. DSM 4201 und DSM 4202 können auch dessen Mutanten und Varianten verwendet werden, soweit sie eben die Verbindung der allgemeinen Formel I herstellen können. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N′-nitro-N-nitroso-guanidin (MNNG) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole, wie Glucose, Lactose oder D-Mannit, sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali- oder Erdalkalimetalle, Eisen, Zink und Mangan als zusätzliche anorganische Salze enthalten.

Die Bildung der Verbindung der allgemeinen Formel I verläuft besonders gut in einer Nährlösung, die Glycerin in Konzentrationen von 0,5 bis 6 %, bevorzugt 2 bis 4 %, sowie Sojamehl in Konzentrationen von 0,1 bis 4 %, bevorzugt 0,5 bis 2 % enthält, jeweils bezogen auf das Gewicht der gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40 °C, vorzugsweise bei etwa 25 bis 30 °C, insbesondere bei 28 bis 30 °C erfolgen. Man kultiviert den Mikroorganismus unter den genannten Bedingungen bis die stationäre Phase erreicht ist, etwa 60 bis 120 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden.

Die Angucyclinone der allgemeinen Formel I liegen sowohl im Mycel als auch in der Kulturbrühe vor. Daher wird zweckmäßig für die Isolierung der Substanz beides aufgearbeitet. Vorteilhaft wird vor der eigentlichen Aufarbeitung das Mycel von der Kulturbrühe getrennt, beispielsweise durch Filtrieren oder Zentrifugieren. Die Verbindung der allgemeinen Formel I kann dann aus dem Überstand bzw. Filtrat isoliert werden, zweckmäßig im pH-Bereich von 2 bis 8, vorzugsweise bei pH-Werten von 5 bis 7. Der Stoffe kann mit herkömmlichen Mitteln, beispielsweise polaren Lösungsmitteln extrahiert werden, z.B. niederen Alkanolen. Es ist jedoch vorteilhaft, die Flüssigkeit über ein Adsorberharz, wie beispielsweise solche auf Polystyrolbasis, zu geben. Die Elution kann dann mit einem polaren Lösungsmittel erfolgen, bevorzugt niedrige Alkanole wie z.B. Methanol, die möglicherweise noch mit Wasser vermischt werden. Aus dem Eluat kann das Lösungsmittel durch Destillation entfernt und der wäßrige, die Angucyclinone enthaltende Rückstand, getrocknet werden.

Die Angucyclinone der allgemeinen Formel I sind farblose amorphe Feststoffe, die gut in Methanol, Aceton, DMSO, Dioxan und Chloroform löslich sind, jedoch nicht in Wasser und Alkanen. Durch basenkatalysierte Acylierung der Hydroxylgruppen mit einen entsprechenden Säureanhydrid gelangt man zu den gewünschten Acyloxyderivaten. Die Verbindungen der allgemeinen Formel I sowie die ($C_1$bis$C_5$)-Acyloxyderivate, bevorzugt ($C_1$bis$C_2$)-Acyloxyderivate, können entsprechend ihrer Stabilität in galenische Zubereitungen eingearbeitet werden. Die antibakterielle und antifungische Wirkung kann im Agar-Diffusionstest in vitro gezeigt werden. Die Angucyclinone weisen außerdem eine starke Wirkung gegen Protozoen, besonders gegen Trichomonas vaginalis auf.

In den folgenden Beispielen wird die Erfindung weitergehend erläutert. Prozentangaben beziehen sich, wie schon in der vorangegangenen Beschreibung, auf das Gewicht. Die in folgenden angegebenen Rf-Werte beziehen sich auf SiIG/UV 254 + 366; 0,25 mm Schichtdicke, Fa. Macherey & Nagel.

## Beispiele

1. a) Herstellung einer Sporensuspension des Produzentenstammes:

100 ml Nährlösung (4 g Hefeextrakt, 10 g Malzextrakt, 4 g Glukose, 1 l Leitungswasser, pH-Wert vor dem Sterilisieren 7,3) in einem 500 ml Erlenmeyerkolben werden mit dem Stamm DSM 4201 und DSM 4202 beimpft und 72 Stunden bei 27 °C und 120 UpM auf der rotierenden Schüttelmaschine inkubiert.

Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 27° C inkubiert. Die nach dieser Zeit entstandenen Sporen eines Kolbens werden mit 500 ml entionisiertem Wasser, das einen Tropfen eines handelsüblichen nichtionischen Tensids (Triton X100, Fa. Serva) enthält, abgeschwemmt, sofort weiterverwendet oder bei -22° C aufbewahrt.

b) Herstellung einer Kultur bzw. Vorkultur des Produzentenstammes im Erlenmeyerkolben.

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (pH 7,2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27° C inkubiert. Die maximale Antibiotikumproduktion ist nach 72 Stunden erreicht. Zum Animpfen von 10 und 100 l Fermentern genügt eine 48 Stunden alte Submerskultur (5 %) aus der gleichen Nährlösung.

2. Herstellung der Angucyclinone

Ein 10 l Fermenter wird unter folgenden Bedingungen betrieben:

| | | |
|---|---|---|
| Nährmedium: | 30 g/l | Glycerin |
| | 2 g/l | Caseinpepton |
| | 1 g/l | $K_2HPO_4$ |
| | 1 g/l | NaCl |
| | 0,5 g/l | $MgSO_4 \cdot H_2O$ |
| | 5 ml/l | Spurenelementlösung |
| Spurenelemente: | 3 g/l | $CaCl_2 \cdot 2H_2O$ |
| | 1 g/l | $FeC_6O_7H_5$ |
| | 0,2 g/l | $MnSO_4$ |
| | 0,1 g/l | $ZnCl_2$ |
| | 0,025 g/l | $CuSO_4 \cdot 5H_2O$ |
| | 0,02 g/l | $Na_2B_4O_7 \cdot 10H_2O$ |
| | 0,004 g/l | $CoCl_2$ |
| | 0,01 g/l | $Na_2MoO_4 \cdot 2H_2O$ |
| Inkubationszeit: | 72 Stunden | |
| Inkubationstemperatur: | 30° C | |
| Rührergeschwindigkeit: | 250 UpM | |
| Belüftung: | 4 l Luft/Min. | |

Durch wiederholte Zugabe weniger Tropfen ethanolischer Polyollösung kann die Schaumentwicklung unterdrückt werden. Das Produktionsmaximum wird nach ca. 70 Stunden (pH = 5,3) erreicht.

3. Isolierung der Angucyclinone

Nach der Fermentation von DSM 4201 und DSM 4202 wird die Kulturbrühe unter Zusatz von 2 % Celite als Filterhilfsmittel filtriert. Das Mycel wird mit Aceton extrahiert, die organische Phase eingedampft und der wäßrige Rückstand zum Kulturfiltrat gegeben. Das Kulturfiltrat wird über ein Adsorberharz auf Polystyrolbasis (XAD2, Fa. Fluka) gegeben. Der Durchlauf wird verworfen und die Angucyclinone mit Methanol/$H_2O$ (80:20) eluiert. Das Eluat wird destilliert. Die Angucyclinone befinden sich im Destillationsrückstand.

4. Isolierung und Charakterisierung der Verbindung

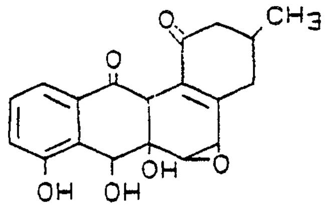

( Ia )

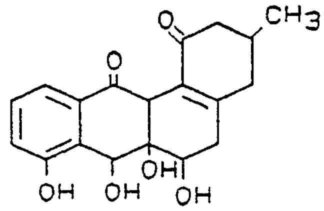

( Ib )

Die Verbindungen Ia und Ib wurden aus dem Kulturfiltrat von Streptomyces DSM 4201 und DSM 4202 gewonnen.

Das Lyophilisat aus einer 100 l Fermentation wurde mit 10 g Kieselgel in wenig Laufmittel suspendiert, auf eine vorbereitete Kieselgel-Säule (10 x 25 cm; Kieselgel 0,04 -0,063 mm) gegeben und mit Chloroform-Methanol (2 l, 20:1; 5 l Gradient bis 9:1) unter Mitteldruck in 2 Fraktionen aufgetrennt:
Fraktion 1: 20 g Ib, Rf = 0,37 (Chloroform-Methanol 9:1, v:v)
mit wenig Ia, Rf = 0,72 (Chloroform-Methanol = 9:1) verunreinigt,
Fraktion 2: 11 g Ib, Rf = 0,37 (Chloroform-Methanol = 9:1, v:v).
Die Fermentation lieferte 20 mg/l Ia und 111 mg/l Ib.

**Verbindung Ib**

Schmp.: 95° C
Rf = 0.37 (Chloroform-Methanol = 9:1, v:v).
IR (KBr): $\nu$ = 3420, 2960, 1680, 1640, 1595, 1470, 1390, 1295, 1078 $cm^{-1}$.
UV (MeOH): $\lambda$ (lg $\epsilon$) = 257 (3,8), 326 (3,2), 353 (3,2) nm.
$^1H$-NMR (200 MHz; DMSO-$d_6$, 37° C): $\delta$ = 9,85 - 9,2 (s; 1H), 7,0-6,8 (m; 3H), 5,05 (s; 1H), 5,00 - 4,65 (s; 1H, H/D), 4,50 - 4,24 (s; 1H, H/D), 3,91 (s; 1H), 3,01 (dd, J = 8,0 Hz; J = 8,0 Hz; 1H), 2,20 - 1,70 (m; 7H), 0,76 (d, J = 5,0 Hz; 3H) ppm.
$^{13}C$-NMR (200 MHz, $CDCl_3$): $\delta$ = 199,8 (C=O), 195,9 (C=O), 157,2 (C), 155,5 (C), 130,9 (C), 129,9 (CH), 128,2 (C), 126,3 (C), 121,8 (CH), 118,7 (CH), 74,5 (C), 66,6 (CH), 63,2 (CH), 47,6 (CH), 45,3 ($CH_2$), 38,9 ($CH_2$), 38,3 ($CH_2$), 29,1 (CH), 20,9 ($CH_3$) ppm.
Fast-atom-bombardment-MS: (positive Ionen) m/e = 345 ($MH^+$, 100 %).
(negative Ionen) m/e = 343 ($M-H^-$, 100%)
EI-MS (70 eV): m/e = 306 (m-$2H_2O$-2H, 49 %; Hochauflösung: 306.0893 entspricht $C_{19}H_{14}O_4$).
Summenformel: $C_{19}H_{20}O_6$ (344.3676 g $mol^{-1}$).

Durch eine zweite säulenchromatographische Trennung der 1. Fraktion, welche genau wie die oben beschriebene durchgeführt wurde, konnten 2 Fraktionen erhalten werden:
Fraktion 1: 5,46 g, Ia Rf = 0,72 (Chloroform-Methanol = 9:1),
Fraktion 3: 458 mg Ib, Rf = 0,37 (Chloroform-Methanol = 9:1).

**Verbindung Ia**

Schmp.: 257° C
Rf = 0,72 (Chloroform-Methanol = 9:1, v:v).
$[\alpha]_D^{20}$ + 28,5° (c = 1,05 $CH_3OH$).
IR (KBr): $\nu$ = 3500-3200, 2960, 2930, 2900, 2870, 1690 (sh), 1675, 1650, 1623, 1580, 1455, 1312, 1300 $cm^{-1}$.
UV ($CHCl_3$:$CH_3OH$ = 1:1): $\lambda$ (lg $\epsilon$) = 258 (3,9), 314 (3,4).

$^1$H-NMR (200 MHz; DMSO-$d_6$, 37 °C): δ = 7,3 - 7,2 (m; 2H), 7,1 - 7,0 (m; 1H), 5,25 - 5,1 (m; 1H, H/D), 5,03 (s, 1H), 3,63 (d, J = 1,5 Hz; 1H), 3,45 (d, J = 4,0 Hz), 3,37 (d, J = 4,0 Hz; 1H), 3,4 - 3,2 (m; 1H, H/D), 2,7 - 2,0 (m; 5H), 0,99 (d, J = 6,0 Hz; 3H) ppm.
$^1$H-NMR (200 MHz, $CDCl_3/CD_3OD$): δ = 7,50 (dd, J = 1,2 Hz, J = 8,0 Hz; 1H), 7,30 (dd, J = 8,0 Hz, J = 8,0 Hz; 1H), 7,08 (dd, J = 1,2 Hz, J = 8,0 Hz; 1H), 5,18 (s; 1H), 3,85 (dd, J = 1,5 Hz, J = 3,0 Hz; 1H), 3,67 (d, J = 4,0 Hz; 1H), 3,44 (d, J = 4,0 Hz; 1H), 2,27 - 2,04 (m; 5H), 1,09 (d, J = 6 Hz; 3H) ppm.
$^{13}$C-NMR (200 MHz, Aceton-$d_6/CD_3OD$): δ = 198,4 (C=O), 197,7 (C=O), 157,5 (C), 153,8 (C), 134,7 (C), 132,7 (C), 130,1 (CH), 127,0 (C), 121,2 (CH), 119,1 (CH), 75,6 (C), 68,8 (CH), 58,6 (CH), 54,1 (CH), 49,9 (CH), 38,4 ($CH_2$), 31,0 ($CH_2$), 30,5 (CH), 21,2 ($CH_3$) ppm.
Fast-atom-bombardment-MS (negative Ionen): m/e = 341 (m-$H_2O$-H).
Summenformel: $C_{19}H_{29}O_7$ (360.3670).

5. Herstellung und Charakterisierung der Acetyl-Derivate der Verbindung Ib

Tri-O-acetyl-Ib:

313 mg (0.91 mmol) Ib wurden einen Tag bei Raumtemperatur in 20 ml Pyridin-Acetanhydrid (= 1:1) gerührt. Das Reaktionsgemisch gab man auf 50 ml Eiswasser und extrahierte die wäßrige Phase nach 30 min. dreimal mit je 50 ml Diethylether. Die vereinigten Extrakte schüttelte man mit 50 ml 2M HCl aus, trocknete diese über Natriumsulfat und verdampfte das Lösungsmittel im Vakuum.

Die säulenchromatographische Trennung erfolgte wie im vorangehenden Beispiel. Sie lieferte ein Hauptprodukt: 221,7 mg Tri-O-acetyl-Ib (52 %).
Schmp.: 95 - 98 ° C.
Rf = 0,98 (Chloroform-methanol = 9:1, v:v).
IR (KBr): ν = 3440, 2950, 1760, 1735, 1690, 1650, 1600, 1425, 1365, 1230, 1188, 1040, 1020 $cm^{-1}$.
UV (MeOH): λ (lg ε) = 253 (3,7), 290 (3,2) nm.
$^1$H-NMR (200 MHz; $CDCl_3$): δ = 7,90 (dd, J = 1,2 Hz, J = 7,8 Hz; 1H), 7,53 (dd, J = 7,8 Hz, J = 8,0 Hz; 1H), 7,32 (dd, J = 1,2 Hz, J = 8,0 Hz; 1H), 6,55 (s, 1H), 4,75 (dd, J = 7,8 Hz, J = 9,0 Hz; 1H), 4,53 (s; 1H), 3,60 (s; 1H, H/D), 2,8 - 1,95 (m; 7H), 2,41 (s; 3H), 2,08 (s; 3H), 2,14 (s; 3H), 1,08 (d, J = 5,5 Hz; 3H) ppm.
$^{13}$C-NMR (200 MHz; $CDCl_3$): δ = 197,9 (C=O), 193,2 (C=O), 171,2 (C=O), 169,9 (C=O), 169,4 (C=O), 154,4 (C), 149,5 (C), 132,2 (C), 130,9 (CH), 128,8 (C), 128,8 (CH), 127,7 (C), 125,6 (CH), 72,98 (C), 69,3 (CH), 64,5 (CH), 47,6 (CH), 45,2 ($CH_2$), 38,6 ($CH_2$), 34,7 ($CH_2$), 29,0 (CH), 20,8 ($2CH_3$), 20,7 ($2CH_3$), ppm.
EI-MS (70 eV): m/e = 470 ($M^+$, 1 %), 368 (M-2Ac-$H_2O$+2H, 1 %).
Summenformel: $C_{22}H_{26}O_9$ (470.3543 g $mol^{-1}$).

Di-O-acetyl-Ib:

800 mg (2.32 mmol) Ib rührte man 2 Tage bei Raumtemperatur in 10 ml Acetanhydrid-Eisessig (= 1:1). Das Reaktionsgemisch wurde auf 100 ml Eiswasser gegossen und nach 2 h dreimal mit je 20 ml Chloroform extrahiert. Die vereinigten organischen Phasen wurden zweimal mit je 20 ml verdünntem Natriumhydrogencarbonat, dann mit je 20 ml Wasser extrahiert, über Natriumsulfat getrocknet und eingedampft. Das Rohprodukt konnte säulenchromatographisch wie oben beschrieben aufgetrennt werden: 463,7 mg Di-O-acetyl-Ib (47 %),
Schmp.: +100 ° C.
Rf = 0,81 (Chloroform-methanol = 9:1, v:v).
IR (KBr): ν = 3440, 2945, 2929, 1760, 1735, 1680, 1650, 1600, 1368, 1230, 1190 $cm^{-1}$.
UV ($CH_3OH$): λ (lg ε) = 220 (3,9), 246 (4,00), 290 (3,1) nm.
$^1$H-NMR (200 MHz; $CDCl_3$): δ = 7,92 (dd, J = 1,2 Hz, J = 7,8 Hz; 1H), 7,53 (dd, J = 7,8 Hz, J = 8,0 Hz; 1H), 7,34 (dd, J = 1,2 Hz, J = 8,0 Hz; 1H), 6,57 (s; 1H), 4,50 (s; 1H), 3,72 (ddd, J = 7,0 Hz, J ≈ 8 Hz, J ≈ 8 Hz: 1H), 3,08 (s; 1H, H/D), 2,8 - 2,5 (s; 1H, H/D), 2,8 - 2,0 (m; 7H), 2,41 (s; 3H), 2,08 (s; 3H), 1,06 (d, J = 6,0 Hz; 3H) ppm.
$^{13}$C-NMR (200 MHz; $CDCl_3$): δ = 197,8 (C=O), 193,9 (C=O), 171,1 (C=O), 171,0 (C=O), 154,8 (C), 149,2 (C), 132,2 (C), 130,99 (CH), 129,2 (C), 128,3 (CH), 127,8 (C), 125,8 (CH), 74,4 (C), 66,7 (CH), 65,1 (CH), 47,2 (CH), 45,3 ($CH_2$), 38,8 ($CH_2$), 37,5 ($CH_2$), 29,1 (CH), 20,9 ($2CH_3$), 20,9 ($CH_3$), 20,8 ($CH_3$) ppm.

EI-MS (70 eV): m/e = 428 ($M^+$, 1 %), 368 (M-Ac-$H_2O$ + H, 22 %), 308 (M-2Ac-2$H_2O$ + 2H, 70 %).
Summenformel: $C_{23}H_{24}O_8$ (428.4509 g $mol^{-1}$).

O-Acetyl-O-methyl-Ib:

390 mg (0.83 mmol) Tri-O-acetyl-Ib wurden in 10 ml $CHCl_3$ gelöst, und mit 15 Tropfen 1M methanolischem Natriumhydroxid 12 h gerührt. Mit 0.1M Salzsäure stellte man den pH-Wert auf 6,5 ein und extrahierte das Reaktionsgemisch dreimal mit je 50 ml Wasser. Die mit 100 ml Toluol versetzte organische Phase wurde im Vakuum eingedampft und man chromatographierte wie oben beschrieben: 165 mg O-Acetyl-O-methyl-Ib (48 %).
Schmp.: 105 °C
Rf = 0.83 (Chloroform-Methanol = 9:1, v:v).
IR (KBr): $\nu$ = 3440, 2950, 2830, 1735, 1680, 1660, 1590, 1469, 1372, 1288, 1245 $cm^{-1}$.
UV (MeOH): $\lambda$ (lg $\epsilon$) = 228 (4,0), 252 (3,9), 323 (3,3) nm.
$^1$H-NMR (200 MHz; $CDCl_3$): $\delta$ = 7,58 (dd, J = 1 Hz, J = 8 Hz; 1H), 7,30 (bs; 1H, H/D), 7,22 (dd, J = 8 Hz, J = 8 Hz; 1H), 6,87 (dd, J = 1Hz; J = 8 Hz; 1H), 5,00 (s; 1H), 4,84 (dd, J = 7 Hz, J = 8,7 Hz; 1H), 4,49 (s; 1H), 3,46 (s; 1H, H/D), 2,8 -2,0 (m; 7H), 2,13 (s; 3H), 1,09 (d, J = 6 Hz; 3H) ppm.
$^{13}$C-NMR (200 MHz; $CDCl_3$): $\delta$ = 199,0 (C=O), 195,1 (C=O), 170,8 (C=O), 155,9 (C), 154,9 (C), 131,5 (C), 130,2 (CH), 128,7 (C), 123,9 (C), 121,4 (CH), 119,6 (CH), 74,4 (C), 71,4 (CH), 68,7(CH), 57,4 (OMe), 48,98 (CH), 45,2 ($CH_2$), 38,8 ($CH_2$), 28,97 (CH), 20,7 ($CH_3$) ppm.
EI-MS (70 eV): m/e = 400 ($M^+$, 3 %).
Summenformel: $C_{22}H_{24}O_7$ (400.4401 $gmol^{-1}$).

Anthron-Derivat aus Ib:

200 mg (0.58 mmol) Ib, 261 mg (1,74 mmol) Natriumjodid und 199 mg (1.12 mmol) p-Toluolsulfonsäure wurden in 10 ml trockenem Acetonitril 5 Tage bei Raumtemperatur gerührt. Man goß das Reaktionsgemisch auf 50 ml Eiswasser und extrahierte nach 30 min dreimal mit je 30 ml Methylenchlorid und die vereinigten organischen Phasen zweimal mit je 50 ml wäßriger Natriumthiosulfatlösung. Die über Natriumsulfat getrocknete organische Phase wurde im Vakuum eingedampft. Die säulenchromatographische Trennung an Kieselgel (0,063 mm, Säule: 2,5 cm x 30 cm) mit n-Hexan-Essigester 95:5 (3 l) über einen Gradienten bis n-Hexan-Essigester 80:20 (41) unter einem Druck von 1 bar ergab 20,2 mg Anthron-Derivat (11 %),
Schmp.: 203 - 219 °C.
Rf = 0.84 (n-Hexan-Essigester 4:1, v:v).
IR (KBr): $\nu$ = 3450, 2945, 2920, 2860, 1620, 1610, 1585 $cm^{-1}$.
UV ($CHCl_3$:MeOH 1:1, V:V): $\lambda$ (lg $\epsilon$) = 358 (3,2), 286 (3,7), 248 (3,9) nm.
$^1$H-NMR (200 MHz; $CDCl_3$): $\delta$ = 12,79 (s; 1H, H/D), 8,57 (d, J = 8,0 Hz; 1H), 7,50 (dd, J = 8,0 Hz, J = 8,0 Hz; 1H), 7,39 (d, J = 8,0 Hz; 1H), 7,03 (dd, J = 1,0 Hz, J = 8,0 Hz; 1H), 6,88 (dd, J = 0,8 Hz, J = 8,0 Hz; 1H), 4,84 (s; 2H), 3,2 - 2,3 (m; 5H), 1,17 (d, J = 6,0 Hz; 3H) ppm.
$^{13}$C-NMR (200 MHz; $CDCl_3$): $\delta$ = 199,9 (C=O), 188,6 (C=O), 162,6 (C), 151,3 (C), 143,4 (C), 143,0 (C), 135,96 (CH), 131,6 (CH), 130,6 (C), 129,3 (C), 128,3 (CH), 119,1 (CH), 115,7 (C), 114,5 (CH), 49,2 ($CH_2$), 39,8 ($CH_2$), 32,9 ($CH_2$), 29,7 (CH), 21,1 ($CH_3$) ppm.
EI-MS (70 eV): m/e = 292 (M, 100 %);
Hochauflösung = 292.1099 entspricht $C_{19}H_{16}O_3$).
Summenformel: $C_{19}H_{16}O_3$ ( 292.3442 g $mol^{-1}$).

**Ansprüche**

1. Eine Verbindung der allgemeinen Formel I

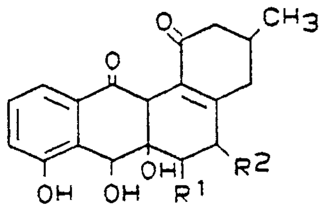

I

in der unabhängig voneinander $R^1$ Hydroxyl und $R^2$ Wasserstoff sein kann oder $R^1$ und $R^2$ zusammen mit den tragenden C-Atomen einen Oxiranring darstellen, sowie die an den in der Formel I aufgeführten Hydroxylgruppen derivatisierten ($C_1$bis$C_5$)-Acyloxyverbindungen.

2. Verfahren zur Herstellung der Verbindung der allgemeinen Formel I sowie deren ($C_1$bis$C_5$)-Acyloxyderivate nach Anspruch 1, dadurch gekennzeichnet, daß

a) Streptomyces spec. DSM 4201 und/oder DSM 4202 oder deren Mutanten und Varianten kultiviert werden bis sich die Verbindung der allgemeinen Formel I in der Kultur anhäuft und

b) gegebenenfalls die Verbindung isoliert und acyliert wird.

3. Verfahren nach Anspruch 2, dadurch gekennzeichnet, daß der Mikroorganismus in einer Nährlösung kultiviert wird, die 0,5 bis 6 % Glycerin sowie 0,1 bis 4 % Sojamehl enthält, jeweils bezogen auf das Gewicht der gesamten Nährlösung.

4. Verfahren nach Anspruch 3, dadurch gekennzeichnet, daß die Nährlösung 2 bis 4 % Glycerin sowie 0,5 bis 2 % Sojamehl enthält.

5. Verfahren nach einem oder mehreren der Ansprüche 2 bis 4, dadurch gekennzeichnet, daß die Kultivierung in einem Temperaturbereich von etwa 18 bis 40 °C durchgeführt wird.

6. Verwendung der Verbindung der allgemeinen Formel I nach Anspruch 1 als therapeutisch wirksamer Stoff.

7. Verwendung nach Anspruch 6, dadurch gekennzeichnet, daß die Verbindung der allgemeinen Formel I als Antibiotikum eingesetzt wird.

8. Verwendung nach Anspruch 7, dadurch gekennzeichnet, daß das Antibiotikum gegen Bakterien und Pilze sowie gegen Protozoen eingesetzt wird.

9. Streptomyces spec. DSM 4201 und DSM 4202 sowie deren Varianten und Mutanten, sofern sie die Verbindung der allgemeinen Formel I nach Anspruch 1 synthetisieren.

Patentansprüche für folgende Vertragsstaaten: ES, GR

1. Verfahren zur Herstellung der Verbindung der allgemeinen Formel I

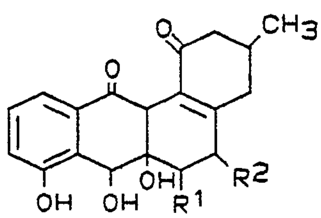

I

in der unabhängig voneinander $R^1$ Hydroxyl und $R^2$ Wasserstoff sein kann oder $R^1$ und $R^2$ zusammen mit den tragenden C-Atomen einen Oxiranring darstellen, sowie die an den in der Formel I aufgeführten Hydroxylgruppen derivatisierten ($C_1$bis$C_5$)-Acyloxyverbindungen, dadurch gekennzeichnet, daß

a) Streptomyces spec. DSM 4201 und/oder DSM 4202 oder deren Mutanten und Varianten kultiviert werden bis sich die Verbindung der allgemeinen Formel I in der Kultur anhäuft und

b) gegebenenfalls die Verbindung isoliert und acyliert wird.

2. Verfahren nach Anspruch 1, dadurch gekennzeichnet, daß der Mikroorganismus in einer Nährlösung kultiviert wird, die 0,5 bis 6 % Glycerin sowie 0,1 bis 4 % Sojamehl enthält, jeweils bezogen auf das Gewicht der gesamten Nährlösung.

3. Verfahren nach Anspruch 3, dadurch gekennzeichnet, daß die Nährlösung 2 bis 4 % Glycerin sowie 0,5 bis 2 % Sojamehl enthält.

4. Verfahren nach einem oder mehreren der Ansprüche 1 bis 3, dadurch gekennzeichnet, daß die Kultivierung in einem Temperaturbereich von etwa 18 bis 40 °C durchgeführt wird.

5. Verwendung der Verbindung der allgemeinen Formel I nach Anspruch 1 als therapeutisch wirksamer Stoff.

6. Verwendung nach Anspruch 5, dadurch gekennzeichnet, daß die Verbindung der allgemeinen Formel I als Antibiotikum eingesetzt wird.

7. Verwendung nach Anspruch 6, dadurch gekennzeichnet, daß das Antibiotikum gegen Bakterien und Pilze sowie gegen Protozoen eingesetzt wird.

8. Streptomyces spec. DSM 4201 und DSM 4202 sowie deren Varianten und Mutanten, sofern sie die Verbindung der allgemeinen Formel I nach Anspruch 1 synthetisieren.